# EUROPEAN PATENT APPLICATION

(11) **EP 0 842 666 A2**
(43) Date of publication of application: **20.05.1998**
(21) Application number: 97203526.5
(22) Date of filing: 12.11.1997
(51) Int. Cl.: A61K 39/40, A61K 38/06

(54) **Combined use of anti-endotoxin synthetic peptides and of anti-endotoxin antibodies for the prophylaxis and treatment of endotoxicosis and septic shock**

(30) Priority: 13.11.1996 IT MI962354
(71) Applicant: BIOSYNTH S.R.L., I-53040 Rapolano Terme (IT)
(72) Inventor: Porro, Massimo, 53040 Rapolano Terme (Siena) (IT)
(74) Representative: De Gregori, Antonella

(57) **Abstract**

Methods and compositions for neutralizing endotoxin and for the prophylaxis and treatment of endotoxicosis and septic shock are disclosed, which comprise the use of peptides specifically binding to the conserved endotoxin structure (Lipid A), and antibodies specifically binding to the antigenic determinants in the endotoxin core structure of different genera of Gram-negative bacteria.

## Description

Shock induced in mammals, particularly humans, by bacterial endotoxins present in the blood and organs (endotoxemia) is also known as septic shock. This physiological condition is a life-threatening situation which occurs in humans following infections by Gram-negative bacteria as a complication of surgery, prolonged hospitalization, accidents and other traumatic events. It is today well recognized that the agent responsible for this disease is bacterial endotoxin, which is a glycolipid antigen present only on the surface of Gram-negative bacteria. This glycolipid is also known as lipo-polysaccharide (LPS) or lipo-oligosaccharide (LOS) depending on the length of the saccharide chain which is covalently bound to the Lipid A (Lip A) structure. Lipid A has the structure of a bis-phosphorylated disaccharide with fatty acids substituting various hydrogen atoms pertaining to the disaccharide hydroxyls. Only Lipid A is responsible for the toxic effects of bacterial endotoxins, a fundamental characteristic being that its molecular structure is conserved between various LPS. When LPS is released into the blood stream by bacteria, specialized cells of the immune system like macrophages and macrocytes are activated by the LPS and several immune mediators are released (Cytokines such as Interleukin-1 and Interleukin-6; Tumor necrosis factor; Gammainterferon).

Furthermore, endotoxin also activates the complement cascade which results in cell lysis of LPS-activated cells, with the consequent release of proteolytic enzymes promoting the release of vasoactive peptides from platelets (bradykinin and histamine). The final result is death of the patient by circulatory shock in 40-60% of the cases within 48 hours from the initial symptoms. So far, there has been no specific cure or therapy available although intravenous injections of corticosteroids such as methylprednisolone are of some benefit.

Polymyxin B is a peptide antibiotic which binds and detoxifies bacterial endotoxins on the basis of its affinity for Lipid A. Polymyxin B can prevent the effects of septic shock when given prophylactically and therapeutically in animal models. However, Polymyxin B is a highly toxic product in vitro and in vivo, a fact which limits its potential use as a therapeutic agent for the treatment of septic shock.

Septic shock can be caused by infection with any bacteria that cause the release of LPS, including Pseudomonas Aeruginosa, Escherichia Coli, Salmonella Typhi, Neisseria Meningitidis, Neisseria Gonorrheae, Bordetella Pertussis, Klebsiella Pneumoniae and the like.

The reasons leading to the reported toxicity of Polymyxin B are not completely understood but are most likely related to the peculiarity of its amino acid composition, specifically for the content of alpha-gamma-diaminobutyric acid (DAB) (49. % w/w of the structure) which is an analog of the amino acid Lysine (reported in literature as able to substitute Lysine in the protein synthesis) and for the presence of D-Phenylalanine, an isomer of the naturally occurring L-Phenylalanine. Other possible reasons, still related to the amino acid composition, could be related to the high stability of Polymyxin B to proteolytic enzymes as well as to the possible binding to cell receptors structurally comparable to the Lipid A of LPS (the gangliosides of the neutral tissues are glycolipids with N,O-acyl (C₁₄-C₁₈) chains very similar to the N,O-acyl chains present in the Lipid A structure).

It is known that certain conformation peptides (endotoxin or SAEP binding peptides) although structurally different from Polymyxin B by virtue of their amino acid composition are capable of binding to the same binding site within the Lipid A of the LOS/LPS as Polymyxin B. These peptides are well tolerated by mammals on the basis of their biodegradability which does not enable them to accumulate within the organs and tissues, in contrast to Polymyxin B. The binding efficiency of certain SAEPs for Lipid A is comparable to the affinity constant value of Polymyxin B. The complex formed when Lipid A or LPS is reacted with these peptides is non-toxic and the natural antigenicity of Lipid A and LPS is maintained. These SAEPs can be monomers, linear polymers, cyclic monomers or cyclic polymers and include those peptides of formula (I) which have amphipathic-polycationic characteristics:

R₁-(A-B-C)ₙ-R (I)

wherein R₁ and R are independently H or an amino acid residue or a fatty acid residue; A is an amino acid selected from the group consisting of Lysine, Arginine and Histidine; B is an amino acid selected from the group consisting of Phenylalanine, Tyrosine and Tryptophan; C is an amino acid selected from the group consisting of Leucine, Isoleucine and Valine; n is a polymerization index from 1 to 100 and preferably from 1 to 10.

It is also known that LPS binding monoclonal antibodies (anti-LPS antibodies) may be prepared which recognize epitopes in the core region of the LPS molecule and are cross-reactive and cross-protective against endotoxicosis caused by Gram-negative bacteria.

These anti-LPS antibodies bind to the antigenic determinants of the LPS core of different genera of Gram-negative bacteria where the core consists essentially of an oligosaccharide region bound to Lipid A but not involving the specific O-carbohydrate region of LPS. Said antibodies:
a) bind to the LPS core structure from at least one species of Gram-negative bacteria and from each of the genera of Escherichia, Salmonella and Pseudomonas LPS; and
b) are effective in treating clinical manifestations of infection in a mammalian host caused by Gram-negative bacteria when administered to the host in a convenient quantity.

### SUMMARY OF THE INVENTION

The present invention is based on the combined use of LPS binding peptides (specific for the Lipid A region) and LPS binding antibodies (specific for the core region), to detoxify LPS in vivo and in vitro. The detoxification of LPS provides an approach to the prophylaxis and treatment of septic shock as well as the removal of LPS from substrates that are prepared for infusion into humans and animals.

Accordingly, it is a primary object of the invention to provide novel prophylactic and therapeutic agents which may be used in the treatment of endotoxicosis and septic shock.

It is also an object of the invention to provide novel pharmaceutical compositions which may be used in the treatment of septic shock.

It is also an object of this invention to provide novel antigenic and non-toxic complexes of Lipid A or LPS with a peptide and an LPS recognizing antibody with different molecular specificities.

It is also an object of this invention to provide a method of producing novel non-toxic Lipid A or LPS antigens.

In particular, it is object of the present invention a pharmaceutical composition which comprises:
(1) an endotoxin binding peptide which is a monomer, linear polymer, cyclic monomer or cyclic polymer of formula:
   (a)

      R₁-(A-B-C)ₙ-R (I)

      wherein R₁ and R are independently H or an amino acid residue or a fatty acid residue; A is an amino acid selected from the group consisting of Lysine, Arginine and Histidine; B is an amino acid selected from the group consisting of Phenylalanine, Tyrosine and Tryptophan; C is an amino acid selected from the group consisting of Leucine, Isoleucine and Valine; n is an integer from 1 to 100;
   (b) (A)n wherein A is Lysine and/or Arginine and n is an integer with a minimum value of 7;
   (c) (AB)m wherein A is Lysine or Arginine and/or B is a hydrophobic amino acid selected from the group consisting of Valine, Leucine, Isoleucine, Tyrosine, Phenylalanine and Tryptophan; m is an integer with a minimum value of 3;
   (d) (ABC)p wherein A is a cationic amino acid which is Lysine and/or Arginine, B and C are hydrophobic amino acids which may be the same or different and are selected from the group consisting of Valine, Leucine, Isoleucine, Tyrosine, Phenylalanine and Tryptophan; p is an integer with a minimum value of 2; and
(2) an antibody specifically binding to the antigenic determinants present in the endotoxin core of different genera of Gram-negative bacteria, where the core consists essentially of Lipid A and of the immediately adjacent core oligosaccharide covalently bound to the Lipid A of the endotoxin, wherein said antibody:
   (a) binds to the endotoxin core from at least one species of Gram-negative bacteria from each kind of endotoxin oligosaccharide core region produced by Escherichia, Salmonella, Pseudomonas, Klebsiella and Neisseria.

It is also an object of the present invention the use of this pharmaceutical composition as a medicament and in particular its use in the prophylaxis and/or treatment of endotoxicosis and septic shock.

Physiological conditions other than septic shock where LPS is the principal cause may be treated by the combined use of peptides and anti-LPS antibodies of this invention. These conditions include, but are not limited to, bacterial meningitis, viral HIV-related infections, pertussis, etc.

These and further objects of the invention will become apparent on reading the following detailed description.

### DETAILED DESCRIPTION OF THE INVENTION

The LPS binding and neutralizing peptides are described in U.S, Patent Nos. 5,358,933 and 5,371,186, and in PCT/Publication No. WO95/03327, the contents of which are incorporated into this description.

Known LPS binding monoclonal antibodies may be produced using procedures described by Kohler and Milstein, Nature, 256:495, 1975. Certain LPS binding antibodies are described in U.S. Patent Nos. 5,057,598 and 5,179,018, in PCT Publication No. WO/92/16624, and in Di Padova et al., Inf. & Immun. 61:9 p3863-3872 (1993), the contents of which are incorporated into this description by reference.

As the binding specificity characteristics are different for anti-LPS peptides and anti-LPS antibodies, the two molecular entities can be administered at the same time in admixture or separately at different times with or without antibiotics, for the prophylaxis and treatment of endotoxicosis and septic shock in humans or mammals in general. The dose of SAEP in humans can vary from 0.1 µg to 2 mg/kg of body weight parenterally or intravenously in a single or multiple dose on a daily basis. The dose of anti-LPS antibodies may vary depending on the severity of the patient's condition. The antibodies can be administered intravenously or parenterally in a single or multiple dose between 0.1 µg and 15 mg/kg of body weight. For in vitro use to detoxify LPS, a dose may be used which is similar to the aforestated dose for in vivo use. The dose may be based on routine experimentation and be varied within and outside the aforestated concentration range, depending on the particular severity of the disease and the condition of the host. Those skilled in pharmacology may ascertain the proper dose using standard procedures.

The SAEPs and the anti-LPS antibodies may be administered intravenously or parenterally using well known pharmaceutical carriers or inert diluents. Oral administration is not preferred because the two molecular entities tend to be degraded by the enzymes of the alimentary tract. Water or isotonic saline solution are preferred diluents for an SAEP or antibody concentration of between 0.01 and 1 mg/ml.

The invention also includes the detoxifying use of the SAEP and anti-LPS antibody in systems containing LPS dispersed in a fluid.

This procedure may be used to detoxify pharmaceuticals such as vaccines, solutions of drugs, injectable nutrient solutions, and the like. The invention further comprises the joint use of the two molecules as additives for fluids which can develop bacterial microbic growth with consequent LPS production. In this respect, the presence of the non-toxic compositions of the invention will detoxify any LPS which is produced following bacterial contamination.

## Claims

1. A pharmaceutical composition which comprises:
(1) an endotoxin binding peptide which is a monomer, linear polymer, cyclic monomer or cyclic polymer of formula:
(a)
R₁-(A-B-C)ₙ-R (I)
wherein R₁ and R are independently H or an amino acid residue or a fatty acid residue; A is an amino acid selected from the group consisting of Lysine, Arginine and Histidine; B is an amino acid selected from the group consisting of Phenylalanine, Tyrosine and Tryptophan; C is an amino acid selected from the group consisting of Leucine, Isoleucine and Valine; n is an integer from 1 to 100;
(b) (A)n wherein A is Lysine and/or Arginine and n is an integer with a minimum value of 7;
(c) (AB)m wherein A is Lysine or Arginine and/or B is a hydrophobic amino acid selected from the group consisting of Valine, Leucine, Isoleucine, Tyrosine, Phenylalanine and Tryptophan; m is an integer with a minimum value of 3;
(d) (ABC)p wherein A is a cationic amino acid which is Lysine and/or Arginine, B and C are hydrophobic amino acids which may be the same or different and are selected from the group consisting of Valine, Leucine, Isoleucine, Tyrosine, Phenylalanine and Tryptophan; p is an integer with a minimum value of 2; and
(2) an antibody specifically binding to the antigenic determinants present in the endotoxin core of different genera of Gram-negative bacteria, where the core consists essentially of Lipid A and of the immediately adjacent core oligosaccharide covalently bound to the Lipid A of the endotoxin, wherein said antibody:
(a) binds to the endotoxin core from at least one species of Gram-negative bacteria from each kind of endotoxin oligosaccharide core region produced by Escherichia, Salmonella, Pseudomonas, Klebsiella and Neisseria.

2. A pharmaceutical composition according to claim 1, wherein, in the peptide of formula (I), n ranges of from 1 to 10.

3. A pharmaceutical composition which comprises:
(1) an endotoxin binding peptide which is a monomer, linear polymer, cyclic monomer or cyclic polymer of formula:
(a)
R₁-(A-B-C)ₙ-R (I)
wherein R₁ and R are independently H or an amino acid residue or a fatty acid residue; A is an amino acid selected from the group consisting of Lysine, Arginine and Histidine; B is an amino acid selected from the group consisting of Phenylalanine, Tyrosine and Tryptophan; C is an amino acid selected from the group consisting of Leucine, Isoleucine and Valine; n is an integer from 1 to 100;
(b) (A)n wherein A is Lysine and/or Arginine and n is an integer with a minimum value of 7;
(c) (AB)m wherein A is Lysine or Arginine and/or B is a hydrophobic amino acid selected from the group consisting of Valine, Leucine, Isoleucine, Tyrosine, Phenylalanine and Tryptophan; m is an integer with a minimum value of 3;
(d) (ABC)p wherein A is a cationic amino acid which is Lysine and/or Arginine, B and C are hydrophobic amino acids which may be the same or different and are selected from the group consisting of Valine, Leucine, Isoleucine, Tyrosine, Phenylalanine and Tryptophan; p is an integer with a minimum value of 2; and
(2) an antibody specifically binding to the antigenic determinants present in the endotoxin core of different genera of Gram-negative bacteria, where the core consists essentially of Lipid A and of the immediately adjacent core oligosaccharide covalently bound to the Lipid A of the endotoxin, wherein said antibody:
(a) binds to the endotoxin core from at least one species of Gram-negative bacteria from each kind of endotoxin oligosaccharide core region produced by Escherichia, Salmonella, Pseudomonas, Klebsiella and Neisseria,
for use as a medicament.

4. A pharmaceutical composition which comprises:
(1) an endotoxin binding peptide which is a monomer, linear polymer, cyclic monomer or cyclic polymer of formula:
(a)
R₁-(A-B-C)ₙ-R (I)
wherein R₁ and R are independently H or an amino acid residue or a fatty acid residue; A is an amino acid selected from the group consisting of Lysine, Arginine and Histidine; B is an amino acid selected from the group consisting of Phenylalanine, Tyrosine and Tryptophan; C is an amino acid selected from the group consisting of Leucine, Isoleucine and Valine; n is an integer from 1 to 100;
(b) (A)n wherein A is Lysine and/or Arginine and n is an integer with a minimum value of 7;
(c) (AB)m wherein A is Lysine or Arginine and/or B is a hydrophobic amino acid selected from the group consisting of Valine, Leucine, Isoleucine, Tyrosine, Phenylalanine and Tryptophan; m is an integer with a minimum value of 3;
(d) (ABC)p wherein A is a cationic amino acid which is Lysine and/or Arginine, B and C are hydrophobic amino acids which may be the same or different and are selected from the group consisting of Valine, Leucine, Isoleucine, Tyrosine, Phenylalanine and Tryptophan; p is an integer with a minimum value of 2; and
(2) an antibody specifically binding to the antigenic determinants present in the endotoxin core of different genera of Gram-negative bacteria, where the core consists essentially of Lipid A and of the immediately adjacent core oligosaccharide covalently bound to the Lipid A of the endotoxin, wherein said antibody:
(a) binds to the endotoxin core from at least one species of Gram-negative bacteria from each kind of endotoxin oligosaccharide core region produced by Escherichia, Salmonella, Pseudomonas, Klebsiella and Neisseria,
for use in the prophylaxis and/or treatment of endotoxicosis and septic shock.

5. A pharmaceutical composition according to claims 3 or 4, wherein, in the peptide of formula (I), n ranges of from 1 to 10.
